# EUROPEAN PATENT SPECIFICATION

(11) **EP 0 622 615 B1**
(45) Date of publication and mention of the grant of the patent: **03.12.1997**
(21) Application number: 94104419.0
(22) Date of filing: 18.03.1994
(51) Int. Cl.: G01F 17/00, G01F 22/02, A61M 5/142

(54) **Method and apparatus for determining the volume of a bellows reservoir**
Verfahren und Vorrichtung zur Bestimmung des Volumens eines Balgreservoirs
Procédé et appareil pour déterminer le volume d'un réservoir à soufflet

(30) Priority: 22.04.1993 SE 9301344
(43) Date of publication of application: 02.11.1994
(73) Proprietor: Siemens-Elema AB, 171 95 Solna 1 (SE)
(72) Inventor: Palmskog, Göran, S-175 43 Järfälla (SE)

(56) References cited:
- WO-A-89/01795
- US-A- 4 360 019
- US-A- 4 395 259
- US-A- 4 486 190
- US-A- 4 557 726
- US-A- 4 871 351

## Description

The present invention relates to a method and a apparatus for determining the volume of a bellows reservoir for medicines in an implantable infusion system.

There is a need for continuous determination of the volume of such a reservoir, on one hand, in order to secure that the rest volume will not be too small before medicine is refilled, as the air which always is present in the reservoir then will be pumped out into the patient, and, on the other, because the pressure in the bellows reservoir decreases as the rest volume becomes less than 2 - 3 ml, which in its turn brings the pump to operate with larger pressure difference which gives reduced volume per pump stroke and cavitation in the pump which is detrimental to medicines like insulin. Further, it is desirable to be able to determine the volume of the bellows reservoir to avoid that the reservoir is filled too much during the filling operation. In the reservoir a depression shall prevail and during the filling medicine is sucked in by this depression. If, however, the reservoir is filled "too much" the prescribed depression will be lost.

The purpose of the present invention is to propose a method and provide an apparatus for continuous determination of the volume of the bellows reservoir during the operation of the infusion system as well as during emptying and filling of its medicine reservoir.

This purpose is obtained with a method according to claim 1 and an apparatus according to claim 3.

According to an advantageous further development of the method according to the invention said predetermined relationship between pressure and volume is determined by simultaneous measurement of the pressure and volume progresses during filling and emptying of the reservoir. With the aid of this relationship volumes corresponding to measured pressure values are determined.

The pressure gauge can preferably be disposed in the filler port to the reservoir and be formed of a piezo-electric gauge.

The unit for determining the volume from the measured pressure with the aid of the relationship between the quantities pressure and volume for the reservoir in question, which relationship is stored in the unit, is preferably arranged in an external equipment, placed outside the patient, output signals delivered by said pressure gauge being transmitted to this unit in telemetric way.

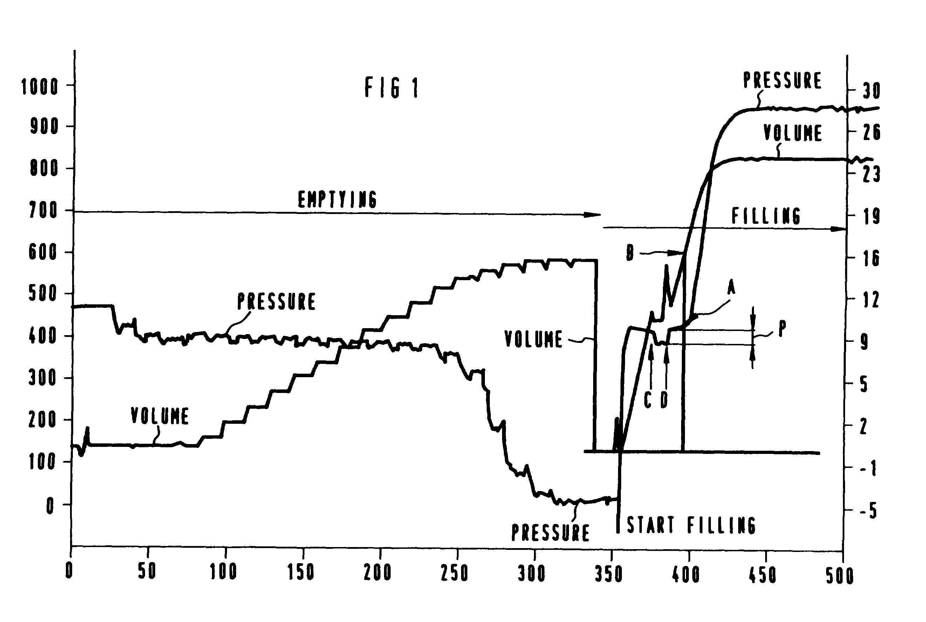

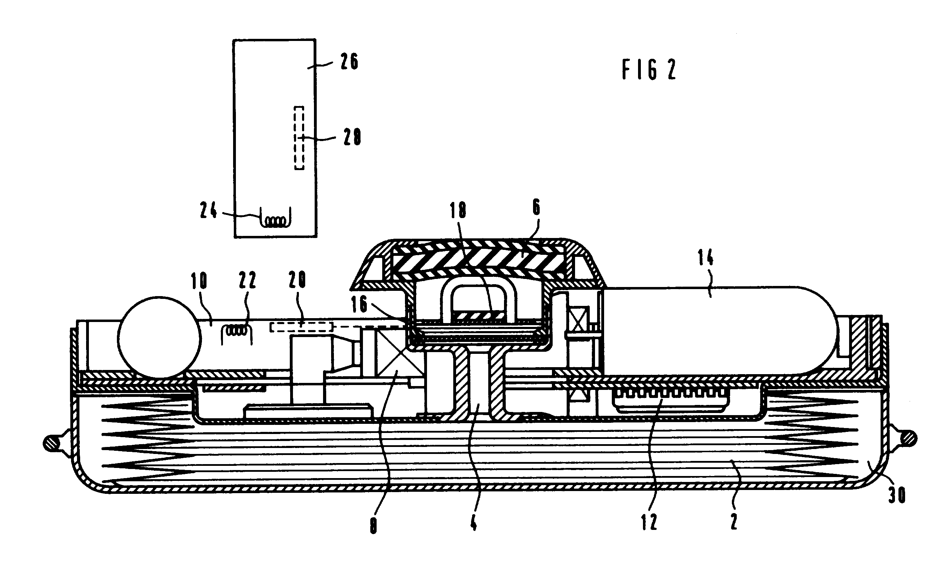

The invention will now be described more in detail with reference to the enclosed drawings on which figure 1 shows typical pressure-volume-curves when emptying residual insulin and filling new insulin in an implantable insulin infusion system, and fig. 2 shows a cross-section through such an infusion system equipped with the apparatus according to the invention.

In fig. 1 typical pressure-volume-curves are shown when emptying residual insulin and filling new insulin in an implantable insulin infusion system of the kind shown in fig. 2. The pressure can then be recorded by a pressure gauge through a cannula introduced through the septum 6 to about the same level as the pressure gauge 18 of the apparatus according to the invention, which gauge is mentioned below, during emptying and filling of carefully determined volumes of insulin. This calibration procedure has been verified on a fifteen reservoirs.

The relationship between pressure and volume can obviously vary between different types of reservoirs. The example illustrated in fig. 1 relates to a bellows reservoir the total volume of which amounts to about 24 ml.

As appears from fig. 1 the pressure curve when emptying the reservoir has a plateau at about 400 mbar. The level for this plateau is depending on the temperature at which the emptying is performed. For an infusion system implanted for animal tests, the temperature then amounting to 38° C, this plateau will be situated at about 700 mbar.

As 2 - 3 ml insulin remains in the reservoir the pressure starts decreasing below the plateau pressure and to empty the last 2 - 3 ml of insulin from the reservoir the pressure must be lowered to less than 100 mbar. This has appeared to be the case for all examined bellows reservoir of the kind in question and for a pressure of 50 mbar still 0,5 - 1 mbar of insulin remains. A pressure gauge recording a pressure threshold of about 600 mbar will for normal body temperature record when the volume of residual insulin drops to 2 - 3 ml, which is suitable in practice. The pump then starts operating against a too low depression and cavitation problems will occur.

Also when filling the reservoir there will be a need for continuous recording of the pressure. Even if the bellows reservoir during filling only must suck in insulin the depression is obviously lost as soon as the bellows wall enters in contact with the bottom of the case. To the right of fig. 1 the pressure-volume-curves are shown during filling of the reservoir. From these curves appear that the specified depression of 450 mbar in this case, at A on the pressure curve, can no longer be maintained as the bellows reservoir is filled to about 17 ml, at B on the volume curve. From point A the pressure is steeply increasing. This rapid pressure increase illustrates the need of a continuous monitoring of the pressure during the filling phase and the apparatus according to the invention is preferably equipped with an alarm which is started as the pressure in the bellows reservoir becomes too high. Moreover, it is an advantage to have such an alarm operative when the pump of the infusion system is in operation. Leakage can namely results in a situation where the bellows bottoms, the depression in the bellows reservoir then being lost with accompanying risk for the patient. The pressure as a function of the volume of the bellows reservoir will progress identically during emptying and filling.

In fig. 2 a cross-section is shown through an infusion system for which the curves shown in fig. 1 have been recorded.

In addition to the bellows reservoir 2 for insulin with its port 4, closed by the septum 6, the system comprises a pump 8, electronics 10, a microprocessor 12 and a battery 14. In the port a filter 16 is disposed and outside the filter the pressure gauge 18 is mounted. The bellows reservoir is arranged in a depression chamber 30.

As pressure gauge 18 several different commercially available piezo-electric gauges can be used. The pressure gauge 18 is connected to an unit 20 contained in the electronics space 10. In the unit 20 the relationship between pressure and volume is stored, which relationship is determined in advance as described above.

At 22 and 24 telemetry coils are shown for communication between the implanted system and an external control and programming unit 26, situated outside the patient. Thus, through the telemetry connection 22, 24 pressure and/or volume values can be continuously transmitted for external recording.

Particulars in the connection of the pressure gauge 18 to the unit 20 and the telemetry coil 22 are not shown as this is well-known techniques to the man skilled in the art.

Instead of locating the unit 20 in the electronics space 10 the unit for determining the volume from the measured pressure can be disposed in the external control and programming unit 26, at 28. The output signal from the pressure gauge 18 is then transmitted directly on telemetric way 22, 24 to the unit 28 in which the previously determined relationship between pressure and volume for the bellows reservoir in question is stored.

In fig. 2 an example is shown of the positioning of the pressure gauge 18 in the port to the bellows reservoir 2. The gauge for sensing the pressure in the bellows reservoir can obviously be arranged in a plurality of different places and by suitable location of the pressure gauge the pressure recording can be used for measuring the fall in pressure across the filter 16 which can be comparatively large after it has been used for some time as a result of stopping up of the filter 16, which in its turn makes both sucking out of residual insulin and filling of new insulin difficult.

In fig. 1 a rapid pressure fall is shown at C which arises as the filling flow is stopped and at D a corresponding rapid pressure increase is shown as the filling flow is started again. This pressure step Δp precisely corresponds to the pressure fall across the filter.

## Claims

1. A method for determining the volume of a bellows reservoir for medicine of an infusion system implantable in a patient, **characterized in** that the pressure is measured in the reservoir and the corresponding volume is determined from a predetermined relationship between pressure and volume.

2. The method according to claim 1, **characterized in** that for determination of said predetermined relationship between pressure and volume simultaneous pressure and volume progresses are measured during filling and/or emptying of the reservoir.

3. An apparatus for determining the volume of a bellows reservoir (2) for medicine of an implantable infusion system, **characterized in** that a pressure gauge (18) is disposed to measure the pressure in the reservoir (2) and in that an unit (20, 28) is arranged to determine a corresponding volume from the measured pressure value by means of a predetermined relationship between pressure and volume.

4. The apparatus according to claim 3, **characterized in** that the pressure gauge (18) is arranged in a port (4) to the reservoir (2).

5. The apparatus according to claims 3 or 4, **characterized in** that the pressure gauge (18) is a piezo-electric gauge.

6. The apparatus according to any of the claims 3 - 5, **characterized in** that the unit (28) for determining the volume from measured pressure is located outside the patient and in that the pressure gauge (18) is disposed to deliver an output signal representing the measured pressure which signal is supplied to said unit in a telemetric way (22, 24).

7. The apparatus according to any of claims 3 - 6, **characterized in** that a pressure gauge is disposed to start an alarm if the pressure exceeds a predetermined level or is less than another, predetermined, lower level.

## Patentansprüche

1. Ein Verfahren zur Bestimmung eines Balgreservoirs für Medikament in einem in einen Patienten implantierbaren Infusionssystems, **dadurch gekennzeichnet,** daß der Druck in dem Reservoir gemessen wird und das korrespondierende Volumen aus einem vorbestimmten Verhältnis zwischen Druck und Volumen bestimmt wird.

2. Das Verfahren nach Anspruch 1, **dadurch gekennzeichnet,** daß zur Bestimmung des vorbestimmten Verhältnisses zwischen Druck und Volumen die Druck- und Volumenverläufe während des Füllens und/oder Entleerens des Reservoirs simultan gemessen werden.

3. Eine Vorrichtung zum Bestimmen des Volumens eines Balgreservoirs (2) für Medikament in einem implantierbaren Infusionssystem, **dadurch gekennzeichnet,** daß ein Druckgeber (18) zum Messen des Druckes in dem Reservoir (2) angeordnet ist und daß eine Einheit (20, 28) zum Bestimmen eines korrespondierenden Volumens aus dem gemessenen Druckwert mittels eines vorbestimmten Verhältnisses zwischen Druck und Volumen angeordnet ist.

4. Die Vorrichtung nach Anspruch 3, **dadurch gekennzeichnet,** daß der Druckgeber (18) in einem Zugang (4) zum Reservoir (2) angeordnet ist.

5. Die Vorrichtung nach Anspruch 3 oder 4, **dadurch gekennzeichnet,** daß der Druckgeber (18) ein piezoelektrischer Geber ist.

6. Die Vorrichtung nach einem der Ansprüche 3 - 5, **dadurch gekennzeichnet,** daß die Einheit (28) zum Bestimmen des Volumens aus dem gemessenen Druck außerhalb des Patienten angeordnet ist und daß der Druckgeber (18) zur Abgabe eines den gemessenen Druck repräsentierenden Ausgangssignals ausgebildet ist, das der Einheit auf telemetrischem Wege (22,24) zugeführt wird.

7. Die Vorrichtung nach einem der Ansprüche 3 - 6, **dadurch gekennzeichnet,** daß der Druckgeber zum Auslösen eines Alarms ausgebildet ist, wenn der Druck ein vorbestimmtes Niveau überschreitet oder ein anderes, vorbestimmtes niedrigeres Niveau unterschreitet.

## Revendications

1. Procédé pour déterminer le volume d'un réservoir à soufflets pour médicament d'un système de perfusion implantable chez un patient, caractérisé en ce que la pression est mesurée dans le réservoir et le volume correspondant est déterminé à partir d'une relation prédéterminée entre pression et volume.

2. Procédé suivant la revendication 1, caractérisé en ce que la détermination de la relation prédéterminée entre pression et volume, les variations simultanées de pression et volume sont mesurées pendant le remplissage et/ou la vidange du réservoir.

3. Dispositif pour déterminer le volume d'un réservoir (2) à soufflets pour médicament d'un système de perfusion implantable, caractérisé en ce que une jauge (18) de pression est disposée pour mesurer la pression dans le réservoir (2) et en ce que une unité (20,28) est agencée pour déterminer un volume correspondant à partir de la valeur de pression mesurée au moyen d'une relation prédéterminée entre pression et volume.

4. Dispositif suivant la revendication 3, caractérisé en ce que la jauge (18) de pression est agencée dans un orifice (4) vers le réservoir (2).

5. Dispositif suivant la revendication 3 ou 4, caractérisé en ce que la jauge (18) de pression est une jauge piézo-électrique.

6. Dispositif suivant l'une quelconque des revendications 3 à 5, caractérisé en ce que l'unité (28) pour déterminer le volume à partir de la pression mesurée est située à l'extérieur du patient et en ce que la jauge (18) de pression est disposée pour envoyer un signal de sortie représentant la pression mesurée, le signal étant envoyé à l'unité par télémétrie (22,24).

7. Dispositif suivant l'une quelconque des revendications 3 à 6, caractérisé en ce qu'une jauge de pression est disposée pour enclencher une alarme si la pression dépasse un niveau prédéterminé ou vient à être inférieur à un autre niveau, prédéterminé, inférieur au précédent.
